Europäisches Patentamt

European Patent Office

Office européen des brevets

(11) Numéro de publication : **0 334 702 B1**

# (12) FASCICULE DE BREVET EUROPEEN

(45) Date de publication du fascicule du brevet :
**17.06.92 Bulletin 92/25**

(51) Int. Cl.$^5$ : **C07C 41/06,** C07C 43/04,
B01J 8/04

(21) Numéro de dépôt : **89400624.6**

(22) Date de dépôt : **06.03.89**

(54) **Procédé de préparation d'un éther alkylique tertiaire par distillation réactive.**

(30) Priorité : **21.03.88 FR 8803776**

(43) Date de publication de la demande :
**27.09.89 Bulletin 89/39**

(45) Mention de la délivrance du brevet :
**17.06.92 Bulletin 92/25**

(84) Etats contractants désignés :
**AT BE DE ES GB IT NL**

(56) Documents cités :
**EP-A- 0 008 860**
**GB-A- 2 096 603**
**US-A- 3 629 478**
**US-A- 4 504 687**
**US-A- 4 540 831**

(72) Inventeur : **Dang Vu, Quang**
**48bis, Boulevard du Général Leclerc**
**F-92200 Neuilly (FR)**
Inventeur : **Amigues, Pierre**
**rue Felin**
**F-69340 Franchevomme (FR)**
Inventeur : **Leonard, Jacques**
**15, rue Anatole France**
**F-95370 Montigny (FR)**
Inventeur : **Gaillard, Jean-Ferdinand**
**rue du Commandant Charcot**
**F-69000 Lyon (FR)**
Inventeur : **Nocca, Jean-Luc**
**7, Boulevard Marcel Pourtout**
**F-92500 Rueil-Malmaison (FR)**

(73) Titulaire : **INSTITUT FRANCAIS DU PETROLE**
**4, Avenue de Bois Préau**
**F-92502 Rueil-Malmaison (FR)**
Titulaire : **ELF FRANCE**
**Tour Elf 2, Place de la Coupole**
**LA DEFENSE 6 - 92400 Courbevoie (FR)**

Jouve, 18, rue Saint-Denis, 75001 PARIS

## Description

La présente invention est relative à un procédé de préparation d'un éther alkylique tertiaire par réaction d'un alcool aliphatique avec un mélange d'hydrocarbures renfermant au moins une iso-oléfine.

Elle est également relative à un appareil dans lequel un tel procédé peut être mis en oeuvre.

La présente invention concerne plus particulièrement la préparation du méthyl tertio-butyl éther (MTBE) à partir d'isobutène et de méthanol, la préparation du méthyl tertio-amyl éther (TAME) à partir d'isopentène et de méthanol et la préparation de MTBE et de TAME à partir d'isobutène, d'isopentène et de méthanol.

Les éthers alkyliques tertiaires, en particulier le méthyl tertio-butyl éther (MTBE) et le méthyl tertio-amyl éther (TAME), constituent des additifs très intéressants pour l'amélioration des qualités des essences, en raison notamment de leurs propriétés antidétonantes.

Il est connu de préparer des éthers alkyliques tertiaires, en particulier le méthyl tertio-butyl éther (MTBE) et le méthyl tertio-amyl éther (TAME) qui sont parmi les éthers les plus courants, en faisant réagir une iso-oléfine généralement contenue dans une fraction d'hydrocarbures avec un alcool aliphatique, par exemple le méthanol, en présence d'un catalyseur acide, par exemple l'acide sulfurique, l'acide fluorhydrique, le chlorure d'aluminium ou le fluorure de bore, ou en présence de matières carbonées contenant des groupes - $SO_3H$, par exemple des charbons sulfonés, des résines phénol-formaldéhyde sulfonées, des polymères coumarone-indène sulfonés ou, de préférence, des résines polystyrène-divinylbenzène sulfonées.

On sait depuis longtemps que la réaction entre le méthanol et les oléfines tertiaires est équilibrée, et qu'il est donc difficile d'obtenir des taux acceptables de conversion des iso-oléfines sans utiliser des excès de méthanol très élevés par rapport à la stoechiométrie ; mais le méthanol forme avec les hydrocarbures légers des azéotropes rendant très difficile sa récupération par des voies classiques comme, par exemple, la distillation azéotropique combinée avec un recyclage au réacteur, tel que décrit dans le brevet FR-B2411881. Le brevet US-A4204077 enseigne une récupération du méthanol par une extraction au solvant tel que l'éthylène glycol. Mais, ces méthodes ne sont applicables qu'au prix d'un investissement couteux et d'une exploitation complexe (dus à la nécessité d'utiliser plusieurs réacteurs et colonnes de distillation pour préparer et séparer l'éther alkylique tertiaire).

Il a alors été proposé une méthode pour convertir en majeure partie les iso-oléfines sans utiliser nécessairement un excès de méthanol : c'est la méthode de la distillation réactive (ou distillation catalytique), qui consiste à effectuer dans la même enceinte la réaction d'éthérification avec un catalyseur et la distillation pour séparer l'éther alkylique tertiaire des autres constituants non convertis au fur et à mesure qu'il se forme (US-A3629478, EP-B0008860, FR-B 2503700).

Le brevet EP-B0008860 propose ainsi d'utiliser une colonne de distillation remplie d'un catalyseur approprié pour la préparation du méthyl tertio-butyl éther (MTBE), dans laquelle le catalyseur agit également comme garnissage pour la distillation, formant ainsi le MTBE et le séparant en même temps des constituants à quatre atomes de carbone. Bien que le procédé décrit dans ce brevet représente un apport technique important dans le domaine de la distillation réactive, le contact entre la phase liquide et le catalyseur semble plus ou moins intermittent du fait de l'effet perturbateur de la phase vapeur.

Le brevet FR-B2503700 propose d'utiliser une série d'étapes catalytiques avec une circulation ascendante vapeur-liquide dans chaque lit catalytique, le catalyseur étant noyé. Mais l'effet de distillation n'est pas aussi important que prévu. De plus, un problème d'hydrodynamique peut se poser : en effet, du fait de la gravité, il ne sera pas toujours aisé pour le liquide de monter à travers chaque lit catalytique.

Le brevet US-A3629478, qui représente l'état de la technique le plus proche, enseigne d'utiliser des plateaux de distillation et de ne disposer le catalyseur, en vrac (c'est-à-dire librement), que dans les descentes de liquide desdits plateaux de distillation, ceci afin d'éviter l'effet perturbateur de la phase vapeur à travers le catalyseur. Mais, la présence du catalyseur dans lesdites descentes crée une perte de charge telle, que le liquide préfère descendre à contre-courant dans les orifices prévus pour la vapeur sur la table de travail de chaque plateau de distillation, plutôt que de passer à travers le catalyseur qui se trouve ainsi mis hors-circuit.

La présente invention permet de remédier aux inconvénients mentionnés précédemment : en effet, au moyen de dispositifs faisant partie intégrante de l'invention, on oblige le liquide de la distillation à passer sur différents lits catalytiques contenus dans une zone de distillation-réaction, c'est-à-dire une zone définie par une enceinte où s'effectuent la réaction et/ou la distillation, on contrôle son temps de séjour à travers ces lits catalytiques et on évite le contact entre le catalyseur et la phase vapeur en aménageant des passages réservés à celle-ci à travers lesdits lits catalytiques. Ainsi, grâce en particulier à un très bon effet de distillation, on obtient des rendements très satisfaisants en éther alkylique tertiaire.

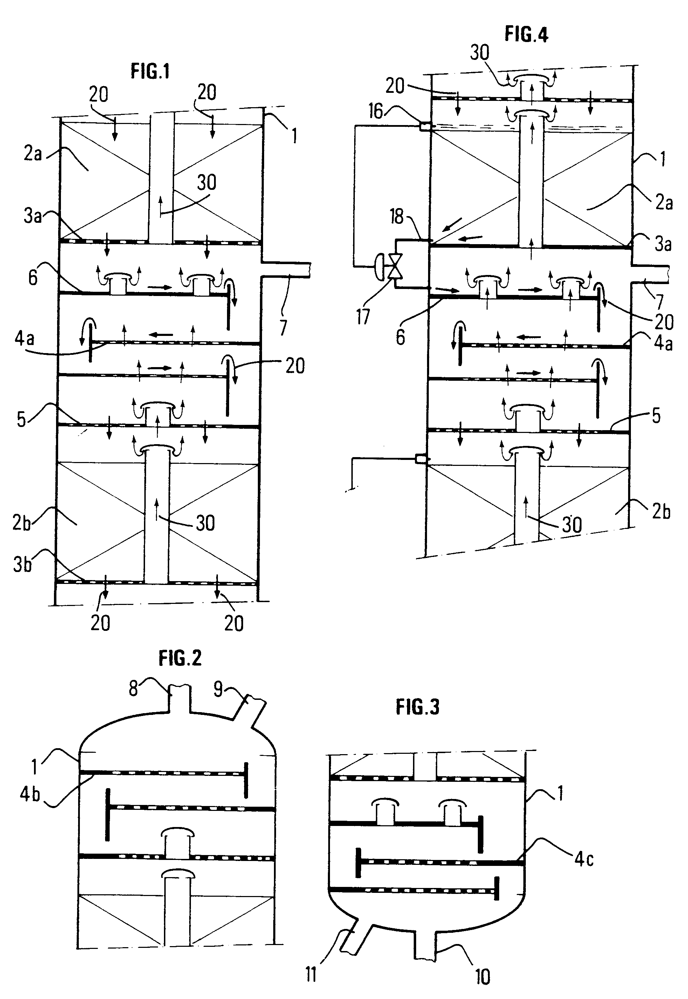

Les figures 1, 2, 3 et 4, données à titre d'exemples, illustrent de manière non limitative l'invention : les figures 1, 2 et 3 concernent respectivement le milieu, le haut et le bas de l'enceinte selon une forme de réalisation de l'invention (où, en particulier, les supports ou fonds définis postérieurement sont perforés) ; la figure 4 concerne le milieu de l'enceinte selon un autre mode de réalisation de l'invention (dans lequel, en particulier, lesdits supports ou fonds ne sont pas perforés, mais étanches).

L'objet de la présente invention est un procédé de préparation d'un éther alkylique tertiaire (ou de plusieurs éthers alkyliques tertiaires) par réaction d'un alcool aliphatique avec un mélange d'hydrocarbures renfermant au moins une iso-oléfine, dans une zone de réaction-distillation (c'est-à-dire une zone où s'effectuent la réaction et/ou la distillation) définie (voir figures 1,2 et 3) par une enceinte (1) de forme sensiblement cylindrique, par exemple verticale, et contenant au moins un catalyseur du type résine sulfonée, par exemple une résine polystyrène-divinylbenzène sulfonée, ce procédé étant caractérisé :

– en ce qu'on introduit la charge de réactifs contenant au moins ledit alcool et au moins ledit mélange d'hydrocarbures dans ladite zone de réaction-distillation qui renferme :

a) au moins deux lits fixes superposés (2a, 2b) et non contigus dudit catalyseur de type résine sulfonée, chacun de ces lits étant disposé sur un support ou fond (3a, 3b), chaque lit de catalyseur et chaque support (ou fond) correspondant étant traversés par au moins un passage libre, sans catalyseur, pour une phase vapeur (par exemple une cheminée ou conduite dont l'extrémité supérieure est de préférence saillante, c'est-à-dire, s'élève ou déborde au-dessus du sommet dudit lit catalytique et dont ladite extrémité peut être de préférence couverte (mais non fermée), empêchant ainsi le liquide présent dans ledit lit de se déverser dans cette cheminée), par exemple situé dans leur partie centrale (les lits catalytiques pouvant avoir des épaisseurs différentes),

b) au moins un plateau de distillation (4a), libre de catalyseur (c'est-à-dire sans catalyseur), situé dans au moins un espace libre se trouvant entre deux lits consécutifs de catalyseur (2a, 2b),

c) au moins un plateau discontinu (c'est-à-dire muni d'une pluralité de discontinuités) de redistribution de liquide (5), libre de catalyseur, situé dans au moins un espace libre se trouvant entre un plateau de distillation et le lit de catalyseur (ou lit catalytique) situé immédiatement en-dessous dudit plateau de distillation, chaque plateau discontinu de redistribution de liquide étant muni en outre d'au moins un passage libre, sans catalyseur, pour une phase vapeur (par exemple une cheminée ou conduite dont l'extrémité supérieure est de préférence saillante, c'est-à-dire, s'élève ou déborde au-dessus du fond dudit plateau de redistribution de liquide et dont ladite extrémité peut être de préférence couverte (mais non fermée), empêchant ainsi le liquide présent sur ledit plateau de se déverser dans cette cheminée), par exemple situé dans sa partie centrale,

– en ce qu'on maintient des conditions de distillation dans ladite zone de réaction-distillation, de façon à avoir une phase liquide et une phase vapeur dans ladite zone,

– en ce qu'on fait circuler la phase liquide du haut vers le bas à travers lesdits lits catalytiques (2a, 2b), le(s)dit(s) plateau(x) de distillation (4a), le(s)dit(s) plateau(x) discontinu(s) de redistribution de liquide (5),

– en ce qu'on maintient une phase continue de liquide dans au moins la partie inférieure de chacun des lits catalytiques par contrôle du niveau de liquide dans chacun desdits lits, c'est-à-dire par contrôle de la résistance à l'écoulement du débit de liquide au bas de chacun desdits lits,

– en ce qu'on fait circuler la phase vapeur du bas vers le haut à travers le(s)dit(s) passage(s) libre(s) desdits lits catalytiques (2a, 2b) (ainsi on ne crée pas de pertes de charge), le(s)dit(s) passage(s) libre(s) dudit (desdits) plateau(x) discontinu(s) de redistribution de liquide (5), le(s)dit(s) plateau(x) de distillation (4a) (cette phase vapeur ascendante n'entrant pas en contact avec le catalyseur dans chacun des lits catalytiques),

– en ce qu'on décharge au sommet (8) de la zone de réaction-distillation une phase vapeur renfermant principalement des hydrocarbures non convertis,

– en ce qu'on soutire à la base (10) de la zone de réaction-distillation une phase liquide renfermant principalement le(s)dit(s) éther(s) alkylique(s) tertiaire(s).

Les discontinuités de chaque plateau de redistribution de liquide (5) sont des orifices pour le passage de la phase liquide, ces orifices étant régulièrement répartis afin de permettre d'arroser uniformément avec du liquide le lit catalytique situé immédiatement en-dessous de chaque plateau de redistribution de liquide.

Tous les plateaux de distillation utilisés dans la présente invention comporte chacun :

– au moins une table de travail discontinue, c'est-à-dire munie de discontinuités pour le passage de la phase vapeur, chaque table de travail étant destinée au brassage et mélangeage des débits liquide et vapeur,

– au moins une descente (ou goulotte), par exemple située au bord de chaque plateau de distillation, pour le conditionnement du liquide et le contrôle de la régularité de son écoulement (descente à travers laquelle va s'écouler le liquide se trouvant auparavant sur la table de travail dudit plateau), et

– au moins un déversoir (ou petite margelle) bordant chaque descente, destiné au maintien d'un certain niveau de liquide sur la table de travail dudit plateau et donc au contrôle de la régularité de l'évacuation du débit liquide de ladite table.

Ces plateaux de distillation peuvent être choisis parmi les plateaux de distillation connus de l'homme de l'art, notamment les plateaux perforés, les plateaux à calottes, les plateaux à clapets.

On peut éventuellement utiliser tout autre dispositif de distillation connu de l'homme du métier, notamment un garnissage inerte ou un ensemble de garnissages inertes (muni d'un support) à la place d'au moins un plateau de distillation.

Selon une forme de réalisation préférentielle de l'invention, on contrôle le niveau de liquide dans chaque lit catalytique (2a, 2b), et donc le temps de contact (entre le liquide et le catalyseur) et de réaction, au moyen, c'est-à-dire par le choix, de perforations régulièrement réparties dans chaque support ou fond correspondant (3a, 3b), perforations par lesquelles s'écoule du liquide contenu dans chaque lit catalytique. Ainsi, on peut maintenir une phase continue de liquide dans chaque lit de catalyseur (2a, 2b) tout en assurant, grâce à chaque support ou fond perforé (3a, 3b), un écoulement sensiblement constant du débit liquide descendant à travers la zone de réaction-distillation. Plus la section totale des perforations d'un fond perforé est faible, plus la hauteur de la phase continue de liquide à l'intérieur du lit correspondant est élevée ; plus le taux de marche est faible, moins la hauteur de liquide à l'intérieur de chaque lit de catalyseur est importante.

Dans la forme de réalisation préférentielle précédente de l'invention, on peut placer (voir figure 1 par exemple), dans chaque espace libre se trouvant entre un plateau de distillation et le lit catalytique situé immédiatement au-dessus dudit plateau de distillation, un plateau de répartition de liquide (6), libre de catalyseur, éventuellement sensiblement incliné, traversé par au moins une conduite ou cheminée pour le passage de la phase vapeur (conduite ou cheminée dont l'extrémité supérieure est de préférence saillante, c'est-à-dire s'élève ou déborde au-dessus du fond du plateau, et dont ladite extrémité peut être couverte (mais non fermée) par une calotte, empêchant ainsi le liquide de se déverser dans cette conduite ou cheminée) ; chaque plateau de répartition de liquide (6) est en outre muni à une extrémité, éventuellement à son extrémité la plus basse s'il est sensiblement incliné, d'au moins un passage libre (qui est, de préférence, une conduite de déversement ou descente bordée d'un déversoir) pour la phase liquide. Chaque plateau de répartition de liquide est disposé de telle manière qu'après être passée préalablement sur ce plateau de répartition de liquide (6), la phase liquide arrive, sur le plateau de distillation situé immédiatement en-dessous dudit plateau de répartition de liquide, le plus loin du déversoir de celui-ci, c'est-à-dire à l'opposé de ce déversoir l'écoulement du liquide sur la table de travail dudit plateau de distillation est ainsi plus régulière et la distillation sur ledit plateau de distillation encore plus efficace.

Selon une autre forme de réalisation préférentielle de l'invention (voir figure 4, donnée à titre d'exemple), on contrôle le niveau de liquide dans chaque lit catalytique (2a, 2b) (et donc la résistance à l'écoulement du débit de liquide au bas de chaque lit) par l'utilisation d'un support ou fond correspondant étanche (3a, 3b), donc non perforé (sur lequel est disposé ledit lit) et d'au moins un conduit d'évacuation (18), qui permet au liquide de s'écouler du bas dudit lit dans la suite de l'enceinte et qui est équipé d'une vanne de réglage (17) asservie à un contrôleur de niveau (16) du liquide contenu dans ledit lit catalytique (ce contrôleur étant par exemple placé vers la partie supérieure dudit lit).

Dans cette forme de réalisation préférentielle, quel que soit le débit de liquide et/ou le taux de marche de l'unité où est mis en oeuvre le procédé, on peut contrôler le niveau de liquide dans chaque lit catalytique et les temps de réaction correspondants. On sait qu'il est en effet nécessaire d'assurer un minimum de temps de contact entre les réactifs et le catalyseur pour convertir l'iso-oléfine et l'alcool aliphatique, mais un temps de contact trop important aura tendance à favoriser les réactions secondaires telles que la dimérisation ou l'oligomérisation des oléfines par exemple. Aussi, le réglage prévu ici permet d'optimiser le rendement et la sélectivité de la transformation.

De manière avantageuse, au moins une partie de la phase vapeur déchargée au sommet (8) de la zone de réaction-distillation peut être condensée (dans un condenseur situé à l'extérieur de l'enceinte (1) définissant ladite zone et non représenté sur les figures), puis renvoyée dans ladite zone, par exemple vers le sommet de ladite zone, sous forme de débit liquide dénommé reflux.

De même, au moins une partie de la phase liquide soutirée à la base (10) de la zone de réaction-distillation peut être vaporisée (par passage dans un rebouilleur situé à l'extérieur de l'enceinte (1) et non représenté sur les figures), puis réintroduite dans ladite zone, par exemple vers la base de ladite zone, sous forme de débit vapeur dénommé vapeur de rebouillage.

Le catalyseur peut être conditionné sous toute forme adéquate, notamment sous forme sensiblement cylindrique, sous forme sensiblement sphérique.

Dans chaque lit catalytique, le catalyseur peut être enfermé dans une ou plusieurs enveloppes perméables au liquide mais imperméables aux particules catalytiques (c'est-à-dire ne laissant pas passer lesdites particules solides de catalyseur), enveloppes constituées par exemple par une toile en tissu, par une toile à base de fils croisés en majeure partie métallique.

Le catalyseur peut au contraire être disposé en vrac, c'est-à-dire librement, à l'intérieur de chaque lit catalytique. Selon un perfectionnement de l'invention, le liquide s'écoulant du lit catalytique placé le plus bas dans la zone de réaction-distillation est alors collecté, soutiré, puis filtré dans au moins un dispositif de filtrage (situé à l'extérieur de l'enceinte (1)) et réintroduit ensuite dans la zone de réaction-distillation entre la position d'où il a été soutiré et la base de ladite zone, éventuellement sur un plateau de distillation placé immédiatement en-dessous du lit catalytique placé le plus bas dans ladite zone. Ainsi, le filtrage permet d'éliminer tous les bris éventuels de catalyseur entraînés par la phase liquide, et, en particulier, les empêche de venir obturer le fond de l'enceinte (1).

L'espace situé entre le sommet de la zone de réaction-distillation (c'est-à-dire le sommet de l'enceinte (1)) et le lit catalytique situé le plus haut dans ladite zone (voir figure 2) peut contenir, de préférence, au moins un plateau de distillation (4b), libre de catalyseur.

L'espace situé entre la base de la zone de réaction-distillation (c'est-à-dire la base de l'enceinte (1)) et le lit catalytique situé le plus bas dans ladite zone (voir figure 3) peut préférentiellement contenir au moins un plateau de distillation (4c), libre de catalyseur.

Un mode de réalisation préférentiel de l'invention (voir figure 1 ou 4) consiste à introduire la charge de réactifs, contenant au moins l'alcool aliphatique et au moins le mélange d'hydrocarbures, dans la zone de réaction-distillation dans une position (7) telle qu'au moins un lit catalytique se trouve au-dessus de ladite position, et, de manière encore plus préférée, telle que le lit catalytique situé le plus bas dans ladite zone se trouve au-dessus de ladite position.

Selon un perfectionnement de l'invention, on peut introduire, en plus de la charge, dudit alcool, pris isolément (c'est-à-dire seul), dans la zone de réaction-distillation, au moins en un point différent du point d'introduction de la charge et, préférentiellement, en tête d'au moins un lit catalytique et, de manière encore plus préférée, en tête de chaque lit catalytique. Ce complément d'alcool, qui favorise la réaction d'éthérification (et permet de réduire la formation éventuelle de dimères), est alors consommé presque totalement au fur et à mesure de son injection et ne passe qu'en petites quantités dans la phase vapeur où une présence trop importante d'alcool pourrait perturber la séparation hydrocarbures-éther(s) alkylique(s) tertiaire(s).

Ce perfectionnement (introduction d'alcool en tête d'au moins un lit catalytique et, préférentiellement, en tête de chaque lit catalytique) est particulièrement intéressant dans le cas où la charge contient, outre ledit alcool aliphatique et ledit mélange d'hydrocarbures, une teneur appréciable d'éther(s) alkylique(s) tertiaire(s) correspondant(s).

La température de l'alcool injecté est de préférence inférieure à sa température d'ébullition.

On maintient en général un rapport de reflux par rapport au distillat (c'est-à-dire un rapport entre le liquide reflué et le liquide soutiré) compris entre 0,1:1 et 20:1, de préférence entre 0,5:1 et 5:1. On opère le plus souvent, à l'intérieur de l'enceinte (1), dans un domaine de pression et de température relativement large : par exemple, de 1 à 30 bars (soit de 100 à 3000 kPa), de préférence de 2 à 20 bars (soit de 200 à 2000 KPa) pour la pression et de 10 à 200°C, de préférence de 40 à 160°C pour la température (dans toute l'enceinte). Chaque lit catalytique utilisé dans la présente invention occupe toute la section circulaire de la zone de réaction-distillation, c'est-à-dire toute la section circulaire de l'enceinte (1).

Sur les figures 1 et 4, données à titre d'exemples, la phase liquide suit préférentiellement le chemin indiqué par les flèches 20 et la phase vapeur suit préférentiellement le chemin indiqué par les flèches 30.

Au fur et à mesure qu'elle descend dans la zone de réaction-distillation, la phase liquide s'enrichit progressivement en éther alkylique tertiaire qui est moins volatil que l'alcool et l'iso-oléfine n'ayant pas encore réagi et que les autres constituants non éthérifiables du mélange d'hydrocarbures. Ainsi la phase liquide soutirée à la base de la zone de réaction-distillation contient principalement de l'éther alkylique tertiaire. Les lits catalytiques selon l'invention travaillent quasi exclusivement en phase liquide : aussi, leur efficacité est très élevée ; la présence d'alcool sur les plateaux de distillation est aussi sensiblement réduite au minimum et la phase vapeur déchargée au sommet (8) de la zone de réaction-distillation ne contient d'alcool non converti que sous forme de traces.

La présente invention a également pour objet (voir figures 1,2 et 3) un appareil (1), de forme sensiblement cylindrique, comportant au moins une conduite (7) pour l'introduction d'une charge, au moins une conduite (8) située au sommet dudit appareil pour le soutirage d'une phase vapeur, au moins une conduite (9) située au voisinage du sommet dudit appareil pour l'alimentation d'un reflux, au moins une conduite (10) située à la base dudit appareil pour le soutirage d'une phase liquide, au moins une conduite (11) située au voisinage de la base dudit appareil pour l'introduction d'une phase au moins partiellement vaporisée, cet appareil étant caractérisé en ce qu'il renferme :

a) au moins deux lits catalytiques fixes, superposés et non contigus (2a,2b), chacun de ces lits étant disposé sur un support ou fond (3a, 3b), chaque lit et chaque support correspondant étant traversés, dans leur partie centrale, par au moins une cheminée ou conduite et occupant toute la section circulaire interne de l'appareil (1),

b) au moins un plateau de distillation (4a), situé dans au moins un espace libre se trouvant entre deux lits catalytiques consécutifs (2a, 2b),

c) au moins un plateau discontinu (c'est-à-dire muni d'une pluralité de discontinuités) de redistribution de liquide (5), situé dans au moins un espace libre se trouvant entre un plateau de distillation et le lit catalytique situé immédiatement en-dessous dudit plateau de distillation, chaque plateau discontinu de redistribution de liquide étant muni en outre d'au moins une cheminée ou conduite (dont l'extrémité supérieure est de préférence saillante, c'est-à-dire s'élève ou déborde au-dessus du fond dudit plateau de redistribution de liquide et dont ladite extrémité est de préférence couverte (mais non fermée) par une calotte) par exemple située dans sa partie centrale.

On peut éventuellement employer en remplacement d'au moins un plateau de distillation tout autre dispositif de distillation connu de l'homme de l'art, notamment un garnissage inerte ou un ensemble de garnissages inertes (muni d'un support).

L'appareil (1) selon la présente invention peut également renfermer au moins un dispositif choisi dans le groupe constitué par :

– au moins un plateau de distillation (4b), situé entre le sommet de l'appareil et le lit catalytique situé le plus haut dans ledit appareil, et

– au moins un plateau de distillation (4c), situé entre la base de l'appareil et le lit catalytique situé le plus bas dans ledit appareil.

L'appareil (1) selon l'invention peut en outre comporter, dans chaque espace libre se trouvant entre un plateau de distillation et le lit catalytique situé immédiatement au-dessus dudit plateau de distillation, un plateau de répartition de liquide (6), éventuellement sensiblement incliné, traversé par au moins une conduite ou cheminée (dont l'extrémité supérieure est de préférence saillante et de préférence couverte par une calotte), et muni à une extrémité, éventuellement à son extrémité la plus basse si il est sensiblement incliné, d'au moins un passage libre (qui est, par exemple, une conduite de déversement bordée d'un déversoir).

Les procédés selon l'invention peuvent être mis en oeuvre dans cet appareil.

L'appareil peut être utilisé, par exemple, pour la préparation du méthyl tertio-butyl éther (MTBE) à partir de méthanol et d'isobutène, pour la préparation du tertio-amyl méthyl éther (TAME) à partir de méthanol et d'isopentène et pour la préparation du MTBE et du TAME à partir de méthanol, d'isobutène et d'iosopentène, en présence d'un catalyseur approprié, tel qu'un catalyseur du type résine sulfonée (par exemple, une résine polystyrène-divinylbenzène sulfonée), ces utilisations n'étant pas limitatives.

<u>Exemple (comparatif)</u>

Une charge, renfermant du méthanol et un mélange de butènes et butanes contenant environ 25 % d'isobutène déjà converti à 75 % en MTBE dans un lit de catalyseur du type résine sulfonée, est introduite dans une enceinte renfermant un certain nombre de lits dudit catalyseur et de plateaux de distillation : selon le procédé de l'invention (et en opérant à une pression sensiblement de l'ordre de 9 bars, à une température comprise entre 65 et 150°C environ et avec un rapport de reflux de l'ordre de 1:1), on peut alors convertir (en MTBE) dans cette enceinte environ 80 % de l'isobutène résiduel en plaçant, en particulier, un plateau de distillation dans chaque espace se trouvant entre deux lits consécutifs dudit catalyseur (lits munis d'une cheminée).

On constate que le nombre de lits dudit catalyseur employés dans le procédé selon l'invention est la moitié du nombre de lits (non munis d'une cheminée) qu'il faut utiliser, pour obtenir le même résultat, dans une colonne catalytique n'utilisant pas l'invention.

**Revendications**

1. Procédé de préparation d'un éther alkylique tertiaire (voir figures 1, 2, 3), par réaction d'un alcool aliphatique avec un mélange d'hydrocarbures renfermant au moins une iso-oléfine, dans une zone de réaction-distillation définie par une enceinte (1) de forme sensiblement cylindrique et contenant au moins un catalyseur du type résine sulfonée, caractérisé :

– en ce qu'on introduit la charge de réactifs contenant au moins ledit alcool et au moins ledit mélange d'hydrocarbures dans ladite zone de réaction-distillation qui renferme :

a) au moins deux lits fixes superposés (2a, 2b) et non contigus dudit catalyseur de type résine sulfonée, chacun de ces lits étant disposé sur un support (3a, 3b), chaque lit de catalyseur et chaque support correspondant occupant toute la section circulaire interne de l'appareil et étant traversés dans leur partie centrale par au moins un passage libre, sans catalyseur, pour une phase vapeur,

b) au moins un plateau de distillation (4a), libre de catalyseur, situé dans au moins un espace libre se trouvant entre deux lits consécutifs de catalyseur (2a, 2b), et

c) au moins un plateau discontinu de redistribution de liquide (5), libre de catalyseur, situé dans au moins un espace libre se trouvant entre un plateau de distillation et le lit catalytique situé immédiatement en-dessous dudit plateau de distillation, chaque plateau discontinu de redistribution de liquide (5) étant muni en outre d'au moins un passage libre, sans catalyseur, pour une phase vapeur,

– en ce qu'on maintient des conditions de distillation dans la zone de réaction-distillation, de façon à avoir une phase liquide et une phase vapeur dans ladite zone,

– en ce qu'on fait circuler la phase liquide du haut vers le bas à travers lesdits lits catalytiques (2a,2b), le(s)dit(s) plateau(x) de distillation (4a), le(s)dit(s) plateau(x) discontinu(s) de redistribution de liquide (5),

– en ce qu'on maintient une phase continue de liquide dans au moins la partie inférieure de chacun des lits catalytiques par contrôle du niveau de liquide dans chacun desdits lits,

– en ce qu'on fait circuler la phase vapeur du bas vers le haut à travers le(s)dit(s) passage(s) libre(s) desdits lits catalytiques (2a,2b), le(s)dit(s) passage(s) libre(s) dudit (desdits) plateau(x) de redistribution de liquide (5), le(s)dit(s) plateau(x) de distillation (4a),

– en ce qu'on décharge au sommet (8) de la zone de réaction-distillation une phase vapeur renfermant principalement des hydrocarbures non convertis, et

– en ce qu'on soutire à la base (10) de la zone de réaction-distillation une phase liquide renfermant principalement ledit éther alkylique tertiaire.

2. Procédé selon la revendication 1 caractérisé en ce qu'on contrôle le niveau de liquide dans chaque lit catalytique au moyen de perforations régulièrement réparties dans chaque support correspondant.

3. Procédé selon la revendication 2 caractérisé en ce que la phase liquide arrive, sur chaque plateau de distillation situé immédiatement en-dessous d'un lit catalytique, à l'opposé du déversoir dudit plateau de distillation après être passée préalablement sur un plateau de répartition de liquide (6), libre de catalyseur, traversé par au moins une conduite pour le passage de la phase vapeur, situé dans chaque espace libre se trouvant entre un plateau de distillation et le lit catalytique situé immédiatement au-dessus dudit plateau de distillation, chaque plateau de répartition de liquide étant en outre muni à une extrémité d'un passage libre pour la phase liquide.

4. Procédé selon la revendication 1 caractérisé en ce qu'on contrôle le niveau de liquide dans chaque lit catalytique (voir figure 4) par l'utilisation d'un support correspondant étanche et d'au moins un conduit (18) d'évacuation équipé d'une vanne de réglage (17), laquelle est asservie à un contrôleur de niveau (16) du liquide contenu dans ledit lit catalytique.

5. Procédé selon l'une des revendications 1 à 4 dans lequel au moins une partie de la phase vapeur déchargée au sommet (8) de la zone de réaction-distillation est condensée, puis renvoyée dans ladite zone sous forme de débit liquide.

6. Procédé selon l'une des revendications 1 à 5 dans lequel au moins une partie de la phase liquide soutirée à la base (10) de la zone de réaction-distillation est vaporisée puis réintroduite dans ladite zone sous forme de débit vapeur.

7. Procédé selon l'une des revendications 1 à 6 caractérisé en ce que le catalyseur est enfermé dans au moins une enveloppe perméable au liquide et imperméable aux particules de catalyseur.

8. Procédé selon l'une des revendications 1 à 6 caractérisé en ce que le catalyseur est disposé en vrac à l'intérieur de chaque lit catalytique, en ce qu'on collecte et soutire le liquide s'écoulant du lit catalytique placé le plus bas dans la zone de réaction-distillation, en ce qu'on filtre ledit liquide soutiré dans un dispositif de filtrage et en ce qu'on réintroduit ledit liquide soutiré et filtré dans la zone de réaction-distillation.

9. Procédé selon l'une des revendications 1 à 8 dans lequel l'espace situé entre le sommet de la zone de réaction-distillation et le lit catalytique situé le plus haut dans ladite zone contient au moins un plateau de distillation (4b), libre de catalyseur.

10. Procédé selon l'une des revendications 1 à 9 dans lequel l'espace situé entre la base de la zone de réaction-distillation et le lit catalytique situé le plus bas dans ladite zone contient au moins un plateau de distillation (4c), libre de catalyseur.

11. Procédé selon l'une des revendications 1 à 10 caractérisé en ce qu'on introduit la charge de réactifs, contenant au moins ledit alcool et au moins ledit mélange d'hydrocarbures, dans la zone de réaction-distillation dans une position (7) telle qu'au moins un lit catalytique se trouve au-dessus de ladite position.

12. Procédé selon l'une des revendications 1 à 11 caractérisé en ce qu'on introduit, en plus de la charge, dudit alcool pris isolément, dans la zone de réaction-distillation au moins en un point différent du point d'introduction de ladite charge et situé en tête d'au moins un lit catalytique.

13. Procédé selon l'une des revendications 1 à 12 dans lequel l'alcool aliphatique est le méthanol et l'iso-oléfine est choisie dans le groupe formé par l'isobutène, l'isopentène et un mélange d'isobutène et d'isopentène.

14. Appareil (1) de forme sensiblement cylindrique (voir figures 1,2,3) comportant au moins une conduite (7) pour l'introduction d'une charge, au moins une conduite (8) située au sommet dudit appareil pour le soutirage d'une phase vapeur, au moins une conduite (9) située au voisinage du sommet dudit appareil pour l'alimentation d'un reflux, au moins une conduite (10) située à la base dudit appareil pour le soutirage d'une phase liquide, au moins une conduite (11) située au voisinage de la base dudit appareil pour l'introduction d'une phase au moins partiellement vaporisée, caractérisé en ce qu'il renferme :

a) au moins deux lits catalytiques fixes, superposés et non contigus (2a, 2b), chacun de ces lits étant disposé sur un support (3a, 3b), chaque lit et chaque support correspondant occupant toute la section circulaire interne de l'appareil et étant traversés dans leur partie centrale par au moins une cheminée,

b) au moins un plateau de distillation (4a), situé dans au moins un espace libre se trouvant entre deux lits catalytiques consécutifs (2a, 2b), et

c) au moins un plateau discontinu de redistribution de liquide (5), situé dans au moins un espace libre se trouvant entre un plateau de distillation et le lit catalytique situé immédiatement en-dessous dudit plateau de distillation, chaque plateau discontinu de redistribution de liquide étant muni en outre d'au moins une conduite.

15. Appareil selon la revendication 14 caractérisé en ce qu'il comporte en outre au moins un dispositif choisi dans le groupe constitué par :

– au moins un plateau de distillation (4b), situé entre le sommet de l'appareil et le lit catalytique situé le plus haut dans ledit appareil,

– au moins un plateau de distillation (4c), situé entre la base de l'appareil et le lit catalytique situé le plus bas dans ledit appareil, et,

– un plateau de répartition de liquide (6), traversé par au moins une cheminée, muni à une extrémité d'au moins un passage libre, et situé dans chaque espace libre se trouvant entre un plateau de distillation et le lit catalytique situé immédiatement au-dessus dudit plateau de distillation.

## Patentansprüche

1. Verfahren zur Herstellung eines tertiären Alkyläthers (siehe Figuren 1,2,3) durch Reaktion eines aliphatischen Alkohols mit einer Kohlenwasserstoffmischung, die mindestens ein Iso-Olefin enthält, in einer Reaktions-Destillationszone, die durch einen im wesentlichen zylindrischen Behälter (1) bestimmt ist und mindestens einen Katalysator vom Typ eines sulfonierten Harzes enthält, dadurch gekennzeichnet,

– daß die Charge der Reagenzen, die mindestens den Alkohol und mindestens die Kohlenwasserstoffmischung enthält, in die Reaktions-Destillationszone eingeführt wird, die beinhaltet:

a) mindestens zwei ortsfeste, übereinander angeordnete und nicht an den Katalysator vom Typ eines sulfonierten Harzes angrenzende Betten (2a,2b), wobei jedes dieser Betten auf einem Träger (3a,3b) angeordnet ist, jedes Katalysatorbett und jeder entsprechende Träger den gesamten, runden, inneren Querschnitt der Vorrichtung einnimmt und in seinem mittleren Bereich mindestens einen freien Durchlaß ohne Katalysator für eine Dampfphase aufweist,

b) mindestens eine Destillationsscheibe (4a), die katalysatorfrei ist und in mindestens einem Freiraum angeordnet ist, der sich zwischen zwei aufeinander folgenden Katalysatorbetten (2a,2b) befindet, und

c) mindestens eine diskontinuierliche Scheibe zur Redistribution von Flüssigkeit (5), die katalysatorfrei ist und in mindestens einem Freiraum angeordnet ist, der sich zwischen einer Destillationsscheibe und dem unmittelbar unterhalb dieser Destillationsscheibe angeordneten Katalysatorbett befindet, wobei jede diskontinuierliche Scheibe zur Redistribution von Flüssigkeit (5) außerdem mit mindestens einem freien Durchlaß ohne Katalysator für eine Dampfphase ausgestattet ist,

– daß die Destillationsbedingungen in der Reaktions-Destillationszone so aufrecht erhalten werden, daß eine flüssige Phase und eine Dampfphase in dieser Zone vorliegen,

– daß die flüssige Phase von oben nach unten durch die katalytischen betten (2a,2b), die Destillationsscheibe/n (4a), die diskontinuierliche/n Scheibe/n zur Redistribution von Flüssigkeit (5) zirkuliert,

– daß eine kontinuierliche Flüssigkeitsphase zumindest in dem unteren Teil jedes katalytischen Bettes durch Regelung des Flüssigkeitsniveaus in diesen Betten aufrecht erhalten wird,

– daß die Dampfphase von unten nach oben durch den/die freien Durchlaß/Durchlässe der katalytischen Betten (2a,2b), den/die freien Durchlaß/Durchlässe der Scheibe/n zur Redistribution der Flüssigkeit (5) und die Destillationsscheibe/n (4a) zirkuliert,

– daß am Kopf (8) der Reaktions-Destillationszone eine Dampfphase entzogen wird, die hauptsächlich nicht umgewandelte Kohlenwasserstoffe enthält, und daß am Fuß (10) der Reaktions-Destillationszone eine flüssige Phase entzogen wird, die hauptsächlich den tertiären Alkyläther enthält.

2. Verfahren nach Anspruch 1, dadurch gekennzeichnet, daß das Flüssigkeitsniveau in jedem katalytischen Bett mit Hilfe von regelmäßig verteilten Perforationen in jedem entsprechenden Träger geregelt wird.

3. Verfahren nach Anspruch 2, dadurch gekennzeichnet, daß die flüssige Phase auf jeder Destillationsscheibe, die unmittelbar unterhalb eines katalytischen Bettes angeordnet ist, auf der dem Überlauf der Destillationsscheibe gegenüberliegenden Seite ankommt, nachdem sie vorher eine Verteilungsscheibe der Flüssigkeit (6) passiert hat, die katalysatorfrei ist, von mindestens einer Führung für den Durchtritt der Dampfphase durchdrungen ist und in jedem Freiraum angeordnet ist, der sich zwischen einer Destillationsscheibe und dem katalytischen Bett befindet, das unmittelbar oberhalb dieser Destillationsscheibe angeordnet ist, wobei jede Verteilungsscheibe der Flüssigkeit außerdem an einem Ende mit einem freien Durchlaß für die flüssige Phase ausgestattet ist.

4. Verfahren nach Anspruch 1, dadurch gekennzeichnet, daß das Flüssigkeitsniveau in jedem katalytischen Bett (siehe Figur 4) durch die Verwendung eines entsprechenden wasserdichten Trägers geregelt wird und durch Verwendung mindestens einer Ausströmführung, die mit einem Regelschieber (17) ausgestattet ist, der durch einen Kontrolleur des Niveaus (16) der in diesem katalytischen Bett enthaltenen Flüssigkeit gesteuert wird.

5. Verfahren nach einem der Ansprüche 1 bis 4, bei dem mindestens ein Teil der am Kopf der Reaktions-Destillationszone entnommenen Dampfphase kondensiert wird und dann in Form flüssiger Durchflußleistung in diese Zone zurückgeschickt wird.

6. Verfahren nach einem der Ansprüche 1 bis 5, bei dem mindestens ein Teil der am Fuß (10) der Reaktions-Destillationszone entzogenen flüssigen Phase verdampft wird und dann in Form von Dampfdurchflußleistung wieder in diese Zone eingeführt wird.

7. Verfahren nach einem der Ansprüche 1 bis 6, dadurch gekennzeichnet, daß der Katalysator von mindestens einem Umschlag umschlossen ist, der für Flüssigkeiten durchlässig und für die Katalysatorpartikel undurchlässig ist.

8. Verfahren nach einem der Ansprüche 1 bis 6, dadurch gekennzeichnet, daß der Katalysator unverpackt, lose im Inneren jedes katalytischen Bettes angeordnet ist, daß die von dem katalytischen Bett, das am weitesten unten in der Reakations-Destillationszone angeordnet ist, ausfließende Flüssigkeit entzogen wird, daß diese entzogene Flüssigkeit in einer Filtervorrichtung gefiltert wird und daß diese entzogene und gefilterte Flüssigkeit wieder in die Reaktions-Destillationszone eingeführt wird.

9. Verfahren nach einem der Ansprüche 1 bis 8, bei dem der zwischen dem Kopf der Reaktions-Destillationszone und dem am weitesten oben in dieser Zone befindliche Raum angeordneten katalytischen Bett mindestens eine Destillationsscheibe (4b) enthält, die frei von Katalysator ist.

10. Verfahren nach einem der Ansprüche 1 bis 9, bei dem der zwischen dem Fuß der Reaktions-Destillationszone und dem am weitesten unten in dieser Zone angeordneten katalytischen Bett befindliche Raum mindestens eine Destillationsscheibe (4c) enthält, die frei von Katalysator ist.

11. Verfahren nach einem der Ansprüche 1 bis 10, dadurch gekennzeichnet, daß die Charge der Reagenzen, die mindestens den Alkohol und mindestens die Kohlenwasserstoffmischung enthält, an einer Position (7) in die Reaktions-Destillationszone eingeführt wird, so daß sich mindestens ein katalytisches Bett oberhalb dieser Position befindet.

12. Verfahren nach einem der Ansprüche 1 bis 11, dadurch gekennzeichnet, daß zusätzlich zu der Charge der Alkohol isoliert an mindestens einem von dem Einführungspunkt der Charge verschiedenen Punkt, der vor mindestens einem katalytischen Bett angeordnet ist, in die Reaktions-Destillationszone eingeführt wird.

13. Verfahren nach einem der Ansprüche 1 bis 12, bei dem der aliphatische Alkohol Methanol ist und das Iso-Olefin aus der Gruppe des Iso-Buten, Iso-Penten und einer Mischung von Iso-Buten und Iso-Penten gewählt ist.

14. Vorrichtung 1 mit im wesentlichen zylindrischer Form (siehe Figuren 1,2,3) mit mindestens einer Zuführung (7) zum Einführen einer Charge, mindestens einer Ableitung (8) zum Entzug einer Dampfphase, die am Kopfes der Vorrichtung angeordnet ist, mindestens einer Zuführung (9) für den Rücklauf, die im Bereich des Kopfes der Vorrichtung angeordnet ist, mindestens einer Abführung (10) für den Entzug einer flüssigen Phase, die im Bereich des Fußes der Vorrichtung angeordnet ist, mindestens einer Zuführung (11) für das Einführen einer zumindest teilweisen Dampfphase, die im Bereich des Fußes der Vorrichtung angeordnet ist, dadurch gekennzeichnet, daß sie beinhaltet:

a) mindestens zwei ortsfeste, übereinandergeordnete und nicht aneinandergrenzende katalytische Betten (2a,2b), wobei jedes dieser Betten auf einem Träger (3a,3b) angeordnet ist und jedes Bett und jeder entsprechende Träger den gesamten, runden, inneren Querschnitt der Vorrichtung einnimmt und in seinem mittleren Bereich von mindestens einem Kamin durchdrungen wird,

b) mindestens eine Destillationscheibe (4a), die in mindestens einem Freiraum angeordnet ist, der sich zwischen zwei aufeinanderfolgenden katalytischen Betten (2a,2b) befindet, und

c) mindestens eine diskontinuierliche Scheibe zur Redistribution von Flüssigkeit (5), die in mindestens einem Freiraum angeordnet ist, der sich zwischen einer Destillationsscheibe und dem katalytischen Bett befindet, das unmittelbar unterhalb dieser Destillationsscheibe angeordnet ist, wobei jede diskontinuierliche Scheibe zur Redistribution von Flüssigkeit außerdem mit mindestens einer Führung ausgestattet ist.

15. Vorrichtung nach Anspruch 14, dadurch gekennzeichnet, daß sie außerdem mindestens eine Vorrichtung aufweist, die aus der folgenden Gruppe gewählt ist:

– mindestens eine Destillationsscheibe (4b), die zwischen dem Kopf der Vorrichtung und dem am weitesten oben in dieser Vorrichtung angeordneten katalytischen Bett angeordnet ist,
– mindestens eine Destillationscheibe (4c), die zwischen dem Fuß der Vorrichtung und dem am weitestens unten in dieser Vorrichtung angeordneten katalytischen Bett angeordnet ist, und
– eine Scheibe zum Verteilen von Flüssigkeit (6), die von mindestens einem Kamin durchdrungen ist, an einem Ende mit mindestens einem freien Durchlaß ausgestattet ist und in jedem Freiraum angeordnet ist, der sich zwischen einer Destillationscheibe und dem unmittelbar oberhalb dieser Destillationsscheibe angeordneten katalytischen Bett befindet.

## Claims

1. A process for manufacturing a tertiary alkyl ether (see figures 1,2,3) by reacting an aliphatic alcohol with a hydrocarbon mixture containing at least one iso-olefin,in a reaction-distillation zone defined by an enclosure (1) of substantially cylindrical shape, containing at least one catalyst of the sulfonated resin type,characterized by the steps of :
– introducing the charge of reactants containing at least said alcohol and at least said hydrocarbon mixture into said reaction-distillation zone, which contains:
    a) at least two superposed and non-contiguous fixed beds (2a,2b) of said catalyst of sulfonated resin type, each of said beds being placed on a support member , (3a,3b) each catalyst bed and each corresponding support member filling the whole internal circular section of the vessel being traversed in their central part by at least one catalyst-free passage-way, for a vapor phase,
    b) at least one catalyst-free distillation tray, 4a located in at least one free space between two consecutive catalyst beds (2a,2b), and
    c) at least one catalyst-free discontinuous tray (5) for liquid redistribution, located in at least one free space between a distillation tray and the catalyst bed just below said distillation tray, each discontinuous liquid redistribution tray (5) being further provided with at least one catalyst-free passage-way, for a vapor phase,
– maintaining distillation conditions in the reaction-distillation zone, so as to have a liquid phase and a vapor phase in said zone,
– downwardly circulating the liquid phase through said catalyst beds (2a,2b), said distillation tray(s) (4a), said discontinuous liquid redistribution tray(s) (5a),

– maintaining a continuous liquid phase in at least the lower part of each catalyst bed by controlling the liquid level in each of said beds,
– upwardly circulating the vapor phase through said free passage-way(s) of said catalyst beds (2a,2b), said free passage-way(s) of said liquid redistribution tray(s), said distillation tray(s) (4a),
– discharging from the top (8) of the reaction-distillation zone a vapor phase mainly containing unconverted hydrocarbons, and
– withdrawing from the bottom (10) of the reaction-distillation zone a liquid phase mainly containing said tertiary alkyl ether.

2. A process according to claim 1, characterized by a control of the liquid level in each catalyst bed by means of regularly distributed perforations in each corresponding support member.

3. A process according to claim 2, characterized in that the liquid phase is supplied onto each distillation tray just below a catalyst bed, on the side opposite to the overflow of said distillation tray, after previous passage over a catalyst-free liquid distribution tray (6), traversed by at least one duct for the passage of the vapor phase, provided in each free space between a distillation tray and the catalyst bed just above said tray, each liquid distribution tray being further provided at one end with a free passage-way for the liquid phase.

4. A process according to claim 1, characterized by a control of the liquid level in each catalyst bed (see figure 4) by using a corresponding tight support member and at least one discharge duct (18) equipped with a control valve (17), monitored by a telltale (17) sensing the liquid level in said catalyst bed.

5. A process according to claim 1, wherein at least a part of the vapor phase discharged from the top (8) of said reaction-distillation zone is condensed, then fed back to said zone as liquid flow.

6. A process according to claim 1, wherein at least a part of the liquid phase withdrawn from the bottom (10) of the reaction-distillation zone is vaporized and then reintroduced into said zone as vapor flow.

7. A process according to claim 1, characterized in that the catalyst is enclosed in at least one clothing permeable to liquid and impermeable to catalyst particles.

8. A process according to claim 1, characterized in that the catalyst is laid in bulk inside each catalyst bed, in that the liquid flowing from the lowermost catalyst bed of the reaction-distillation zone is collected and withdrawn and in that said withdrawn liquid is filtered in a filtering device and then reintroduced in the reaction-distillation zone.

9. A process according to claim 1, wherein the space between the top of the reaction-distillation zone and the uppermost catalyst bed of said zone contains at least one catalyst-free distillation tray (4b).

10. A process according to claim 1, wherein the space between the bottom of the reaction-distillation zone and the lowermost catalyst of said zone contains at least one catalyst-free distillation tray (4c).

11. A process according to claim 1, characterized in that the charge of reactants, containing at least said alcohol and at least said hydrocarbon mixture, is introduced into the reaction-distillation zone at such a level (7) that at least one catalyst bed is above said level.

12. A process according to claim 1, characterized by the separate introduction, in addition to the charge, of said alcohol into said reaction-distillation zone through at least one intake port, different from that used for introducing said charge and placed at the top of at least one catalyst bed.

13. A process according to claim 1, wherein the aliphatic alcohol is methanol and the iso-olefin is selected from the group formed of isobutene, isopentene and an isobutene-isopentene mixture.

14. An apparatus (1) of substantially cylindrical shape (see figure 1,2,3) comprising at least one line (7) for introducing a charge, at least one line (8) at the top of the apparatus for withdrawning a vapor phase, at least one line (9) in the vicinity of the top of said apparatus for a reflux feed, at least one line (10) at the bottom of said apparatus for withdrawing a liquid phase, at least one line (11) in the vicinity of the bottom of said apparatus for introducing an at least partially vaporized phase, characterized in that it contains :

a) at least two superposed non-contiguous fixed catalyst beds (2a,2b), each of said beds being placed on a perforated support member (3a,3b), each bed and each corresponding support member filling the whole internal circular section of the vessel being traversed in their central part by at least one funnel,

b) at least one distillation tray (4a), placed in at least one free space between two consecutive catalyst beds (2a,2b), and

c) at least one discontinuous liquid redistribution tray (5), placed in at least one free space between a distillation tray and the catalyst bed just below said distillation tray, each discontinuous liquid redistribution tray being further provided with at least one duct.

15. An apparatus according to claim 14 , characterized in that it further comprises at least one device selected from the following :

– at least one distillation tray (4a), placed between the top of the apparatus and the uppermost catalyst bed of said apparatus,

– at least one distillation tray (4c) placed between the bottom of the apparatus and the lowermost catalyst bed of said apparatus, and

– a liquid distribution tray (6), traversed by at least one funnel,provided at one end with at least one free passageway, and placed in each free space between a distillation tray and the catalyst bed just above said distillation tray.

FIG.1

FIG.4

FIG.2

FIG.3